# EUROPEAN PATENT APPLICATION

(11) **EP 1 430 916 A1**
(43) Date of publication of application: **23.06.2004**
(21) Application number: 03258034.2
(22) Date of filing: 19.12.2003
(51) Int. Cl.: A61L 27/18, A61L 27/58

(54) **Crosslinked alkyd polyesters for medical applications**

(30) Priority: 20.12.2002 US 325768
(71) Applicant: ETHICON, INC., Somerville, NJ 08876 (US)
(72) Inventor: Nathan, Aruna, Bridgewater, NJ 08807 (US)
(74) Representative: Mercer, Christopher Paul

(57) **Abstract**

The present invention is directed to synthetic, biodegradable, biocompatible polymers that are the reaction product of a polybasic acid or derivative thereof, a monogylceride, and a hydrophilic polyol, where the polymers contain a crosslinkable region, and to medical devices and compositions.

## Description

### FIELD OF THE INVENTION

The present invention relates to biodegradable, biocompatible, crosslinked alkyd polyesters and blends thereof that may be used to produce medical devices and compositions.

### BACKGROUND OF THE INVENTION

Both natural and synthetic polymers, including homopolymers and copolymers, which are both biocompatible and degradable *in vivo* are known for use in the manufacture of medical devices that are implanted in body tissue and degrade over time. Examples of such medical devices include suture anchor devices, sutures, staples, surgical tacks, clips, plates and screws, drug delivery devices, adhesion prevention films and foams, and tissue adhesives.

Natural polymers may include catgut, cellulose derivatives and collagen. Natural polymers typically degrade by an enzymatic degradation process in the body.

Synthetic polymers may include aliphatic polyesters, polyanhydrides and poly(orthoester)s. Synthetic degradable polymers typically degrade by a hydrolytic mechanism. Such synthetic degradable polymers include homopolymers, such as poly(glycolide), poly(lactide), poly(ε-caprolactone), poly(trimethylene carbonate) and poly(p-dioxanone), and copolymers, such as poly(lactide-co-glycolide), poly(ε-caprolactone-co-glycolide), and poly(glycolide-co-trimethylene carbonate). The polymers may be statistically random copolymers, segmented copolymers, block copolymers or graft copolymers.

Alkyd-type polyesters prepared by the polycondensation of a polyol, polyacid and fatty acid are used in the coating industry in a variety of products, including chemical resins, enamels, varnishes and paints. These polyesters also are used in the food industry to make texturized oils and emulsions for use as fat substitutes.

While much progress has been made in the field of polymeric biomaterials, further developments must be made in order for such biomaterials to be used optimally in the body. There is a need for polymers for use in drug delivery, tissue engineering and medical devices that are biodegradable and biocompatible and that can be crosslinked to form hydrogels. Such hydrogels could be used for delivery of sensitive drugs such as proteins and oligonucleotides, cell encapsulation and delivery, coatings on medical devices, wound dressings and films for surgical adhesion prevention.

### SUMMARY OF THE INVENTION

The present invention is directed to synthetic, biodegradable, biocompatible crosslinkable polymers comprising the reaction product of a polybasic acid or derivative thereof, a monoglyceride, and a hydrophilic polyol, the polymer having at least one crosslinkable region, and to medical devices and compositions containing such polymers.

### DETAILED DESCRIPTION OF THE INVENTION

Alkyd polymers have been prepared by several known methods. For example, alkyd-type polymers were prepared by Van Bemmelen (*J. Prakt. Chem.,* **69** (1856) 84) by condensing succinic anhydride with glycerol. In the "Fatty Acid" method (see Parkyn, et al. *Polyesters* (1967), Iliffe Books, London, Vol. 2 and Patton, In: *Alkyd Resins Technology,* Wiley-Interscience New York (1962)), a fatty acid, a polyol and an anhydride are mixed together and allowed to react. The "Fatty Acid-Monoglyceride" method includes a first step of esterifying the fatty acid with glycerol and, when the first reaction is complete, adding an acid anhydride. The reaction mixture then is heated and the polymerization reaction takes place. In the "Oil-Monoglyceride" method, an oil is reacted with glycerol to form a mixture of mono-, di-, and triglycerides. This mixture then is polymerized by reacting with an acid anhydride.

The synthetic, biodegradable, biocompatible crosslinkable polymers described in the present invention comprise the reaction product of a polybasic acid or derivative thereof, a monoglyceride, and a hydrophilic polyol, the polymer having at least one crosslinkable region. These polymers may be classified as crosslinkable alkyd polyesters. Preferably, the crosslinkable polymers utilized in the present invention are prepared by the polycondensation of a polybasic acid or derivative thereof and a monoglyceride, wherein the monoglyceride comprises reactive hydroxy groups and fatty acid groups. The expected hydrolysis byproducts are glycerol, dicarboxylic acid(s), and fatty acid(s), all of which are biocompatible. Preferably, the polymers utilized in the present invention will have a number average molecular weight between about 1,000 g/mole and about 100,000 g/mole, as determined by gel permeation chromatography. The polymers comprise an aliphatic polyester backbone with pendant fatty acid ester groups.

Hydrophilic polyols that can be used to prepare the crosslinkable polymers include, without limitation, glycols, polyglycerols, polyglycerol esters, glycerol, sugars and sugar alcohols. Glycerol is a preferred hydrophilic polyhydric alcohol due to its abundance and cost.

Monoglycerides that may be used to prepare crosslinkable polymers utilized in the present invention include, without limitation, monostearoyl glycerol, monopalmitoyl glycerol, monomyrisitoyl glycerol, monocaproyl glycerol, monodecanoyl glycerol, monolauroyl glycerol, monolinoleoyl glycerol, monooleoyl glycerol, and combinations thereof. Preferred monoglycerides include monostearoyl glycerol, monopalmitoyl glycerol, monomyrisitoyl glycerol, monolinoleoyl glycerol and monooleoyl glycerol.

Polybasic acids that can be used include natural multifunctional carboxylic acids, such as succinic, glutaric, adipic, pimelic, suberic, and sebacic acids; hydroxy acids, such as diglycolic, malic, tartaric and citric acids; and unsaturated acids, such as fumaric, maleic, citraconic and itaconic acids. Polybasic acid derivatives include anhydrides, such as succinic anhydride, diglycolic anhydride, glutaric anhydride and maleic anhydride, mixed anhydrides, esters, activated esters and acid halides. The multifunctional carboxylic acids listed above are preferred.

The crosslinkable polymers described in the present invention have at least one crosslinkable region. The crosslinkable regions of the crosslinkable polymers may interact to form a three-dimensional crosslinked polymeric structures.

In one embodiment, the crosslinkable region may be part of the alkyd polyester polymer backbone or side chain. Unsaturated bonds, for example double bonds, may provide the crosslinkable region and may be introduced by using monoglycerides or polybasic acids containing at least one double bond. Suitable unsaturated polybasic acids include fumaric, maleic, citraconic and itaconic acids.

Alternatively, multifunctional polybasic acids or hydrophilic polyols, may provide the crosslinkable region and may be introduced to the alkyd polyester polymer backbone by the incorporation of multifunctional polybasic acids such as malic, tartaric, and citric acid, or multifunctional hydrophilic polyols, such as glycerol, polygylcerols, sugars and sugar alcohols to the polymer backbone.

In yet another embodiment, the crosslinkable region may comprise crosslinkable end groups on the alkyd polyester polymer backbone. These crosslinkable end groups include acrylates, diacrylates, oligoacrylates, methacrylates, dimethacrylates and oligomethoacrylates. The crosslinkable end groups are created by end-capping the alkyd polyester polymer with crosslinkable end-cap groups. For example, end-capping the polymers with acryloyl chloride or methacryloyl chloride, will yield acrylate ester or methacrylate ester end groups, respectively. Subsequently, these end groups, if located on two alkyd polyester polymer chains, may be reacted together to form a crosslink between the two polymers.

In preparing the crosslinkable polymers utilized in the present invention, the particular chemical and mechanical properties required of the polymer for a particular use must be considered. For example, changing the chemical composition can vary the physical and mechanical properties, including absorption times. Copolymers can be prepared by using mixtures of diols, triol, hydrophilic polyols, diacids, triacids, and different monoalkanoyl glycerides to match a desired set of properties.

One skilled in the art, once having the benefit of the disclosure herein, will be able to ascertain particular properties of the polymers required for particular purposes, and readily prepare polymers that provide such properties.

The polymerization of the crosslinkable alkyd polyester preferably is performed under melt polycondensation conditions in the presence of an organometallic catalyst at elevated temperatures. The organometallic catalyst preferably is a tin-based catalyst e.g. stannous octoate. The catalyst preferably will be present in the mixture at a molar ratio of polyol and polycarboxylic acid to catalyst in the range of from about 15,000/1 to 80,000/1. The reaction preferably is performed at a temperature no less than about 120°C. Higher polymerization temperatures may lead to further increases in the molecular weight of the copolymer, which may be desirable for numerous applications. The exact reaction conditions chosen will depend on numerous factors, including the properties of the polymer desired, the viscosity of the reaction mixture, and melting temperature of the polymer. The preferred reaction conditions of temperature, time and pressure can be readily determined by assessing these and other factors.

Generally, the reaction mixture will be maintained at about 180°C. The polymerization reaction can be allowed to proceed at this temperature until the desired molecular weight and percent conversion is achieved for the copolymer, which typically will take from about 15 minutes to 24 hours. Increasing the reaction temperature generally decreases the reaction time needed to achieve a particular molecular weight.

In another embodiment, copolymers of crosslinkable alkyd polyesters can be prepared by forming an crosslinkable alkyd polyester prepolymer polymerized under melt polycondensation conditions, then adding at least one lactone monomer or lactone prepolymer. The mixture then would be subjected to the desired conditions of temperature and time to copolymerize the prepolymer with the lactone monomers.

The molecular weight of the prepolymer, as well as its composition, can be varied depending on the desired characteristic that the prepolymer is to impart to the copolymer. Those skilled in the art will recognize that the alkyd polyester prepolymers described herein can also be made from mixtures of more than one diol or dicarboxylic acid.

The alkyd polyester polymers of the present invention are crosslinkable. Crosslinking may either be chemical or physical. Chemically crosslinked polymer chains are connected by covalent bonds, which can be formed by reactive groups contained on the polymers, the addition of crosslinking enhancers and/or irradiation (such as gamma-irradiation). Physical crosslinking on the other hand connects the polymer chains through non-covalent bonds such as van der Waals interactions, hydrogen bonding or hydrophobic interactions. In particular, crosslinking can be used to control the water swellability of the crosslinkable polymer.

The crosslinkable regions are preferably polymerizable by photoinitiation by free radical generation, most preferably in the visible or long wavelength ultraviolet radiation. The preferred crosslinkable regions are end groups on the alkyd polysters comprised of acrylates, diacrylates, oligoacrylates, methacrylates, dimethacrylates, oligomethoacrylates, or other biologically acceptable photopolymerizable groups.

Useful photoinitiators are those which initiate, by free radical generation, polymerization of the crosslinkable regions without cytotoxicity and within a short time frame, preferably minutes, and most preferably seconds. Preferred dyes as initiators for long wave length ultraviolet (LWUV) or visible light initiation are ethyl eosin, 2,2-dimethoxy-2-phenyl acetophenone, other acetophenone derivatives, and camphorquinone. Crosslinking may be initiated among crosslinkable regions by a light activated free-radical polymerization initiator such as 2,2-dimethoxy-2-phenyl acetophenone, other acetophenone derivatives, and camphorquinone. In other cases, crosslinking is initiated among crosslinkable regions by a light-activated free-radical polymerization initiator such as 2,2-dimethoxy-2-phenylacetophenone or a combination of ethyl eosin and triethanol amine (0.001 to 0.1M), for example.

The choice of the photoinitiator is largely dependent on the photopolymerizable regions. For example, if the crosslinkable region includes at least one carbon-carbon double bond, light absorption by the dye can cause the dye to assume a triplet state, the triplet state subsequently reacting with the amine to form a free radical which initiates polymerization. Preferred dyes for use with these materials include eosin dye and initiators such as 2,2-dimethyl-2-phenylacetophenone, 2-methoxy-2-phenylacetophenone, and camphorquinone. Using such initiators, crosslinked polymers may be prepared in situ by LWUV light or laser light.

Initiation of crosslinking is accomplished by irradiation with light at a wavelength of between about 200-700 nm, most preferably in the long wavelength ultraviolet range or visible range, 320 nm or higher, most preferably about 514 nm or 365 nm.

There are several photooxidizable and photoreductible dyes that may be used to initiate crosslinking. These include acridine dyes, for example, acriblarine; thiazine dyes, for example, thionine; xanthine dyes, for example, rose bengal; and phenazine dyes, for example, methylene blue. These are used with cocatalysis such as amines, for example, triethanolamine; sulphur compounds; heterocycles, for example, imidazole; enolates; organometallics; and other compounds, such as N-phenyl glycine. Other initiators include camphorquinones and acetophenone derivatives.

As an alternative to photoinitiation of crosslinking, thermal crosslinking initiator systems may be used. Thermal initiators may be selected to allow polymerization to be initiated at a desired temperature. At times it may be desired to use a high temperature to initiate polymerization such as during a molding process. For many medical uses it will be desired to use systems that will initiate free radical polymerization at physiological temperatures include, for example, potassium persulfate, with or without tetramethyl ethylenediamine; benzoylperoxide, with or without triethanolamine; and ammonium persulfate with sodium bisulfite.

Yet another initiation chemistry that may be used besides photoinitiation is water and amine initiation schemes with isocyanate or isothiocyanate containing end groups used as the crosslinkable regions.

The crosslinked polymers (including copolymers) and blends (hereinafter polymers) can be used for many medical applications including, but not limited to the prevention of surgical and tissue adhesions, tissue coatings, sealants and in tissue engineering applications.

A preferred application is a method of reducing formation of adhesions after a surgical procedure in a patient. The method includes coating damaged tissue surfaces in a patient with an aqueous solution of a light-sensitive free-radical polymerization initiator and a solution containing the crosslinkable alkyd polyester. The coated tissue surfaces are exposed to light sufficient to polymerize the crosslinkable region. The light-sensitive free-radical polymerization initiator may be a single compound (e.g., 2,2- dimethoxy-2-phenyl acetophenone) or a combination of a dye and a cocatalyst (e.g., ethyl eosin and triethanol amine).

Additionally, the crosslinked polymers can be used to form hydrogels which are three dimensional networks of hydrophilic polymers in which a large amount of water is present. In general the amount of water present in a hydrogel is at least 20 weight percent of the total weight of the dry polymer. The most characteristic property of these hydrogels is that it swells in the presence of water and shrinks in the absence of water. The extent of swelling (equilibrium water content) is determined by the nature (mainly the hydrophilicity) of the polymer chains and the crosslinking density.

The kinetics of hydrogel swelling is limited by the diffusion of water through the outer layers of the dried hydrogel. Therefore, while hydrogels swell to a large extent in water, the time it takes to reach equilibrium swelling may be significant depending on the size and shape of the hydrogel. To reduce the amount of time it takes for a hydrogel to reach equilibrium, hydrogel foams may be used. Hydrogels foams may be made by crosslinking polymers in the presence of gas bubbles. The hydrogels foams prepared with macroscopic gas cells will have an open celled structure similar to sponges except that the pore size will generally be an order of magnitude larger.

Hydrogels may be used as wound dressings materials, since the crosslinked hydrogels are durable, non-antigenic, and permeable to water vapor and metabolites, while securely covering the wound to prevent bacterial infection. Hydrogels may also be used for coatings in general and medical coatings in particular. The hydrogel coatings may provide a smooth slippery surface and prevent bacterial colonization on the surface of the medical instrument. For example hydrogels may be used as coatings on urinary catheter surfaces to improve its biocompatibility. Hydrogels may also be used in a variety of applications where the mechanical swelling of the hydrogel is useful such as in catheters as a blend component with a biocompatable elastomer. Additionally, hydrogels could be used for drug delivery or immobilization of enzyme substrates or cell encapsulization.

The crosslinking step to form crosslinked structures can be performed in a variety of ways. For example the crosslinkable polymers may be crosslinked while being synthesized, such as by utilizing multifunctional monomers or oligomers. However, crosslinking at other times is also advantageous. For example crosslinking may be performed during the manufacture of a device such by adding a thermal initiator to the polymer prior to injection molding a device. Additionally, crosslinking of a polymerizable region with a photoinitiator may be performed during stereolithography to form devices. As previously discussed photoinitiation may be used *in vivo* to crosslink the polymers of the present invention for various wound treatments such as adhesion prevention and wound sealing. Coating may also be applied to devices and crosslinked *in situ* to form films that will conform to the surface of the device.

One of the beneficial properties of the crosslinkable alkyd polyesters of this invention is that the ester linkages are hydrolytically unstable, and therefore the polymer is biodegradable because it readily breaks down into small segments when exposed to moist body tissue. In this regard, while it is envisioned that co-reactants could be incorporated into the reaction mixture of the polybasic acid and the diol for the formation of the alkyd polyester, it is preferable that the reaction mixture does not contain a concentration of any co-reactant which would render the subsequently prepared polymer nondegradable. Preferably, the reaction mixture is substantially free of any such co-reactants if the resulting polymer is rendered nondegradable.

In one embodiment of the invention, the crosslinkable alkyd polyesters of the present invention can be used as a pharmaceutical carrier in a drug delivery matrix. The variety of therapeutic agents that can be used in conjunction with the crosslinkable polymers of the invention is vast. In general, therapeutic agents which may be administered via pharmaceutical compositions of the invention include, without limitation, antiinfectives, such as antibiotics and antiviral agents; analgesics and analgesic combinations; anorexics; antihelmintics; antiarthritics; antiasthmatic agents; anticonvulsants; antidepressants; antidiuretic agents; antidiarrheals; antihistamines; antiinflammatory agents; antimigraine preparations; antinauseants; antineoplastics; antiparkinsonism drugs; antipruritics; antipsychotics; antipyretics, antispasmodics; anticholinergics; sympathomimetics; xanthine derivatives; cardiovascular preparations including calcium channel blockers and beta-blockers such as pindolol and antiarrhythmics; antihypertensives; diuretics; vasodilators, including general coronary, peripheral and cerebral; central nervous system stimulants; cough and cold preparations, including decongestants; hormones, such as estradiol and other steroids, including corticosteroids; hypnotics; immunosuppressives; muscle relaxants; parasympatholytics; psychostimulants; sedatives; tranquilizers; naturally derived or genetically engineered proteins, polysaccharides, glycoproteins, or lipoproteins; oligonucleotides, antibodies, antigens, cholinergics, chemotherapeutics, hemostatics, clot dissolving agents, radioactive agents and cystostatics.

Rapamycin, risperidone, and erythropoietin are several bioactive agents that may be used in drug delivery matrices of the present invention.

The drug delivery matrix may be administered in any suitable dosage form such as oral, parenteral, subcutaneously as an implant, vaginally or as a suppository. The therapeutic agent may be present as a liquid, a finely divided solid, or any other appropriate physical form. Typically, but optionally, the matrix will include one or more additives, such as, but not limited to, nontoxic auxiliary substances such as diluents, carriers, excipients, stabilizers or the like. Other suitable additives may be formulated with the crosslinked polymer and pharmaceutically active agent or compound.

The amount of therapeutic agent will be dependent upon the particular drug employed and medical condition being treated. Typically, the amount of drug represents about 0.001% to about 70%, more typically about 0.001% to about 50%, most typically about 0.001 % to about 20% by weight of the matrix.

The quantity and type of crosslinkable alkyd polyester incorporated into the parenteral will vary depending on the release profile desired and the amount of drug employed. The product may contain blends of polyesters to provide the desired release profile or consistency to a given formulation.

The crosslinkable alkyd polyester, upon contact with body fluids including blood or the like, undergoes gradual degradation, mainly through hydrolysis, with concomitant release of the dispersed drug for a sustained or extended period, as compared to the release from an isotonic saline solution. This can result in prolonged delivery, e.g. over about 1 to about 2,000 hours, preferably about 2 to about 800 hours) of effective amounts, e.g. 0.0001 mg/kg/hour to 10 mg/kg/hour) of the drug. This dosage form can be administered as is necessary depending on the subject being treated, the severity of the affliction, the judgment of the prescribing physician, and the like.

Individual formulations of drugs and crosslinkable alkyd polyester may be tested in appropriate *in vitro* and *in vivo* models to achieve the desired drug release profiles. For example, a drug could be formulated with a crosslinkable alkyd polyester and orally administered to an animal. The drug release profile could then be monitored by appropriate means, such as by taking blood samples at specific times and assaying the samples for drug concentration. Following this or similar procedures, those skilled in the art will be able to formulate a variety of formulations.

In a further embodiment of the present invention the polymers and blends thereof can be used in tissue engineering applications, e.g. as supports for cells. Appropriate tissue scaffolding structures are known in the art, such as the prosthetic articular cartilage described in U.S. Pat. No. 5,306,311, the porous biodegradable scaffolding described in WO 94/25079, and the prevascularized implants described in WO 93/08850. Methods of seeding and/or culturing cells in tissue scaffoldings are also known in the art such as those methods disclosed in EPO 422 209 B1, WO 88/03785, WO 90/12604 and WO 95/33821.

In another embodiment, the polymer may be used to prepare coatings for application to a medical device. Such devices may include, without limitation, sutures, meshes, tissue engineering scaffolds, pins, clips, staples, sheets, foams, anchors, screws, plates, films, suture knot clips, pins, clamps, hooks, buttons, snaps, nails, endoscopic instruments, bone substitutes, prosthesis, intrauterine devices, vascular implants or supports, e.g. stents or grafts, or combinations thereof, vertebral discs, extracorporeal tubing for kidney and heart-lung machines and artificial skin. The exact formulation of the coating, including the selection of the solvent suitable for the particular polymer used, additional coating components and concentration of the respective coating components, as well as methods of applying the coating to the device and concentration of coating on the particular medical device, all will be ascertained readily by those skilled in the art once having the benefit of this disclosure.

In one embodiment, a coating containing the polymer may be applied to a surface of a surgical article to enhance the lubricity of the coated surface. The polymer may be applied as a coating using conventional techniques. For example, the polymer may be solubilized in a dilute solution of a volatile organic solvent, such as acetone, methanol, ethyl acetate or toluene, and then the article can be immersed in the solution to coat its surface. Once the surface is coated, the surgical article can be removed from the solution where it can be dried at an elevated temperature until the solvent and any residual reactants are removed.

Although it is contemplated that numerous surgical articles can be coated with the crosslinkable alkyd polyesters of this invention to improve the surface properties of the article, the preferred surgical articles are surgical sutures and needles. The most preferred surgical article is a suture, most preferably attached to a needle. Preferably, the suture is a synthetic degradable suture. These sutures are derived, for example, from homopolymers and copolymers of lactone monomers such as glycolide, lactide, including L-lactide D-lactide, meso-lactide and rac-lactide, ε-caprolactone, *p*-dioxanone, 1,4-dioxanone, 1,4-dioxepan-2-one, 1,5-dioxepan-2-one and trimethylene carbonate. The preferred suture is a braided multifilament suture composed of polyglycolide or poly(glycolide-co-lactide).

The amount of coating polymer to be applied on the surface of a braided suture can be readily determined empirically, and will depend on the particular copolymer and suture chosen. Ideally, the amount of coating copolymer applied to the surface of the suture may range from about 0.5 to about 30 percent of the weight of the coated suture, more preferably from about 1.0 to about 20 weight percent, most preferably from 1 to about 5 weight percent. If the amount of coating on the suture were greater than about 30 weight percent, then it may increase the risk that the coating may flake off when the suture is passed through tissue.

Sutures coated with the polymers of this invention are desirable because they have a more slippery feel, thus making it easier for the surgeon to slide a knot down the suture to the site of surgical trauma. In addition, the suture is more pliable, and therefore is easier for the surgeon to manipulate during use. These advantages are exhibited in comparison to sutures which do not have their surfaces coated with the polymer of this invention.

In another embodiment of the present invention when the article is a surgical needle, the amount of coating applied to the surface of the article is an amount that creates a layer with a thickness ranging preferably between about 2 to about 20 microns on the needle, more preferably about 4 to about 8 microns. If the amount of coating on the needle were such that the thickness of the coating layer was greater than about 20 microns, or if the thickness was less than about 2 microns, then the desired performance of the needle as it is passed through tissue may not be achieved.

In yet another embodiment, the medical device comprises a bone replacement material comprising the crosslinkable alkyd polyester and an inorganic filler. The organic filler may be selected from alpha-tricalcium phosphate, beta-tricalcium phosphate, calcium carbonate, barium carbonate, calcium sulfate, barium sulfate, hydroxyapatite, and mixtures thereof. In certain embodiments the inorganic filler comprises a polymorph of calcium phosphate. Preferably, the inorganic filler is hydroxyapatite. The bone replacement materials may further comprise a therapeutic agent in a therapeutically effective amount, such a growth factor, to facilitate growth of bone tissue. Furthermore, the bone replacement material may comprise a biologically derived substance selected from the group consisting of demineralized bone, platelet rich plasma, bone marrow aspirate and bone fragments. The relative amounts of crosslinkable alkyd polyester and inorganic filler may be determined readily by one skilled in the art by routine experimentation after having the benefit of this disclosure.

The examples set forth below are for illustration purposes only, and are not intended to limit the scope of the claimed invention in any way. Numerous additional embodiments within the scope and spirit of the invention will become readily apparent to those skilled in the art.

In the examples below, the synthesized crosslinkable polymers were characterized via differential scanning calorimetry (DSC), gel permeation chromatography (GPC), and nuclear magnetic resonance (NMR) spectroscopy. DSC measurements were performed on a 2920 Modulated Differential Scanning Calorimeter from TA Instruments using aluminum sample pans and sample weights of 5-10 mg. Samples were heated from room temperature to 100°C at 10°C/minute; quenched to -40°C at 30°C/minute followed by heating to 100°C at 10°C/minute. For GPC, a Waters System with Millennium 32 Software and a 410 Refractive Index Detector were used. Molecular weights were determined relative to polystyrene standards using THF as the solvent. Proton NMR was obtained in deuterated chloroform on a 400MHz NMR spectrometer using Varian software.

### Example 1: Synthesis of polymer containing monooleoyl glyceride and maleic anhydride

142.6 grams of monoleoyl glycerol was added to a dry 250 milliliter, single neck, round bottom flask. A stir bar was added and a nitrogen inlet adapter was attached. The reaction flask was placed in a room temperature oil bath and a nitrogen gas blanket was started. The flask was heated to 140°C, and 39.2 grams of maleic anhydride was added. The temperature was raised to 190°C and maintained for 3 hours. After 3 hours the flask was removed from the oil bath to cool to room temperature. The polymer was a pale yellow, viscous liquid.

GPC measurement determined a number average molecular weight of 1383, and a weight average molecular weight of 6435.

### Example 2: Synthesis of polymer containing monooleoyl glyceride and maleic anhydride and 5 mol percent PEG400

40.1 grams of monooleoyl glycerol and 5.00 grams of PEG400 were added to a dry 100 milliliter, single neck, round bottom flask. A stir bar was added and a nitrogen inlet adapter was attached. The reaction flask was placed into a room temperature oil bath and a nitrogen blanket was applied. The oil bath temperature was raised to 140°C. Once at 140°C, 12.2 grams of maleic anhydride was added. The temperature was raised to 180°C and maintained for 7 hours at 180°C. The flask was removed from the oil bath and allowed to cool to room temperature. The polymer was a pale yellow, viscous liquid. GPC measurement determined a number average molecular weight of 1122, and a weight average molecular weight of 5647.

### Example 3: Synthesis of polymer containing monooleoyl glyceride and maleic anhydride and 25 mol percent PEG400

17.8 grams of monooleoyl glycerol and 20.0 grams of PEG400 were added to a dry 100 milliliter, single neck, round bottom flask. A stir bar was added and a nitrogen inlet adapter was attached. The reaction flask was placed into a room temperature oil bath and a nitrogen blanket was applied. The oil bath temperature was raised to 140°C. Once at 140°C, 9.8 grams of maleic anhydride was added. The temperature was raised to 180°C and maintained for 7 hours at 180°C. The flask was removed from the oil bath and allowed to cool to room temperature. The polymer was a pale yellow, viscous liquid. GPC measurement determined a number average molecular weight of 1230, and a weight average molecular weight of 4481.

### Example 4: Synthesis of polymer containing monooleoyl glyceride, maleic anhydride and 45 mol percent PEG400

3.6 grams of monooleoyl glycerol and 36.0 grams of PEG400 were added to a dry 100 milliliter, single neck, round bottom flask. A stir bar was added and a nitrogen inlet adapter was attached. The reaction flask was placed into a room temperature oil bath and a nitrogen blanket was applied. The oil bath temperature was raised to 140°C. Once at 140°C, 9.8 grams of maleic anhydride was added. The temperature was raised to 180°C and maintained for 7 hours at 180°C. The flask was removed from the oil bath and allowed to cool to room temperature. The polymer was a pale yellow, viscous liquid.

GPC measurement determined a number average molecular weight of 1305, and a weight average molecular weight of 3521.

### Example 5: Synthesis of polymer containing monooleoyl glyceride, maleic anhydride and 5 mol percent PEG600

32.1 grams of monooleoyl glycerol and 6.0 grams of PEG600 were added to a dry 100 milliliter, single neck, round bottom flask. A stir bar was added and a nitrogen inlet adapter was attached. The reaction flask was placed into a room temperature oil bath and a nitrogen blanket was applied. The oil bath temperature was raised to 140°C. Once at 140°C, 9.8 grams of maleic anhydride was added. The temperature was raised to 180°C and maintained for 7 hours at 180°C. The flask was removed from the oil bath and allowed to cool to room temperature. The polymer was a pale yellow, viscous liquid.

GPC measurement determined a number average molecular weight of 1165, and a weight average molecular weight of 5667.

### Example 6: Synthesis of polymer containing monooleoyl glyceride, maleic anhydride and 10 mol percent PEG600

28.5 grams of monooleoyl glycerol and 12.0 grams of PEG600 were added to a dry 100 milliliter, single neck, round bottom flask. A stir bar was added and a nitrogen inlet adapter was attached. The reaction flask was placed into a room temperature oil bath and a nitrogen blanket was applied. The oil bath temperature was raised to 140°C. Once at 140°C, 9.8 grams of maleic anhydride was added. The temperature was raised to 180°C and maintained for 7 hours at 180°C. The flask was removed from the oil bath and allowed to cool to room temperature. The polymer was a pale yellow, viscous liquid.

GPC measurement determined a number average molecular weight of 1202, and a weight average molecular weight of 3601.

### Example 7: Synthesis of polymer containing monooleoyl glyceride, maleic anhydride and 20 mol percent PEG600

21.4 grams of monooleoyl glycerol and 24.0 grams of PEG600 were added to a dry 100 milliliter, single neck, round bottom flask. A stir bar was added and a nitrogen inlet adapter was attached. The reaction flask was placed into a room temperature oil bath and a nitrogen blanket was applied. The oil bath temperature was raised to 140°C. Once at 140°C, 9.81 gms of maleic anhydride was added. The temperature was raised to 180°C and maintained for 7 hours at 180°C. The flask was removed from the oil bath and allowed to cool to room temperature. The polymer was a pale yellow, viscous liquid.

GPC measurement determined a number average molecular weight of 1245, and a weight average molecular weight of 3197.

### Example 8: Synthesis of polymer containing monooleoyl glyceride, maleic anhydride and 40 mol percent PEG600

5.9 grams of monooleoyl glycerol and 40.0 grams of PEG600 were added to a dry 100 milliliter, single neck, round bottom flask. A stir bar was added and a nitrogen inlet adapter was attached. The reaction flask was placed into a room temperature oil bath and a nitrogen blanket was applied. The oil bath temperature was raised to 140°C. Once at 140°C, 8.2 grams of maleic anhydride was added. The temperature was raised to 180°C and maintained for 7 hours at 180°C. The flask was removed from the oil bath and allowed to cool to room temperature. The polymer was a pale yellow, viscous liquid.

GPC measurement determined a number average molecular weight of 1455, and a weight average molecular weight of 3692.

### Example 9: Synthesis of polymer containing monooleoyl glyceride, maleic anhydride and 10 mol percent PEG1000

89.1 grams of monooleoyl glycerol and 62.5 grams of PEG1000 were added to a dry 250 milliliter, single neck, round bottom flask. A stir bar was added and a nitrogen inlet adapter was attached. The reaction flask was placed into a room temperature oil bath and a nitrogen blanket was applied. The oil bath temperature was raised to 140°C. Once at 140°C, 30.6 gms of maleic anhydride was added. The temperature was raised to 180°C and maintained for 7 hours at 180°C. The flask was removed from the oil bath and allowed to cool to room temperature. The polymer was a pale yellow, viscous liquid.

GPC measurement determined a number average molecular weight of 1474, and a weight average molecular weight of 4112.

### Example 10: Synthesis of polymer containing monooleoyl glyceride, succinic anhydride and 25 mol percent PEG400

49.0 grams of monooleoyl glycerol and 50.0 grams of PEG400 were added to a dry 250 milliliter, single neck, round bottom flask. A stir bar was added and a nitrogen inlet adapter was attached. The reaction flask was placed into a room temperature oil bath and a nitrogen blanket was applied. The oil bath temperature was raised to 140°C. Once at 140°C, 25.0 grams of maleic anhydride was added. The temperature was raised to 180°C and maintained for 24 hours at 180°C. The flask was removed from the oil bath and allowed to cool to room temperature. The polymer was a pale yellow, viscous liquid.

GPC measurement determined a number average molecular weight of 948, and a weight average molecular weight of 2276.

### Example 11: Acrylate endcapping of polymer containing monooleoyl glyceride, succinic anhydride and 25 mol percent PEG400

25.3 grams of polymer described in Example 10, 75 milliliters methylene chloride, and 4.0 grams of triethylamine were added to a three-necked 300 milliliter round bottom flask and equipped with a glass stirrer with teflon paddle, septum, N₂ inlet/outlet needles, and thermometer. Meanwhile, in the glove box, 3.6 grams acryloyl chloride were weighed into a syringe and the needle was stoppered using a rubber stopper. The methylene chloride solution was chilled to 0°C using a wet ice/NaCl slush bath with stirring. The acryloyl chloride was added dropwise while the temperature was between 2 and 7°C. The slush bath was removed and the light yellow suspension was allowed to warm to slowly to room temperature. 2 milliliters of ethanol were added to the solution and let stir for 1h to react with any excess acryloyl chloride. The stirring was stopped and the reaction flask was stoppered and stored in the refrigerator overnight.

The triethylamine hydrochloride salt was removed by vacuum filtration and the filtercake was washed twice with 25 milliliters of cold methylene chloride. The filtercake was added to a tared aluminum pan and vacuum dried at room temperature to constant weight. The methylene chloride solution was transferred to a 500mL separatory funnel and washed twice with an equal volume of 1.0 M HCl and twice with an equal volume of brine. The organic layer was dried over magnesium sulfate. The magnesium sulfate was removed by vacuum filtration over celite and the methylene chloride was removed by rotoevaporation. The oil was allowed to cool to room temperature and vacuum dried to constant weight in a vacuum oven at 80°C.

### Example 12: Thermal curing of polymer containing monooleoyl glyceride, maleic anhydride and PEG

2 grams of polymer containing monooleoyl glyceride, maleic anhydride and PEG 400 (25 mol%) synthesized following the procedure of Example 3, and 1 gram of PEG diacrylate (Mn = 575) were weighed into a scintillation vial. To this was added 1 gram of a solution containing ascorbic acid (0.1M) and ammonium persulfate (0.1M). The solution was stirred, poured into an aluminum pan and cured at 60°C under nitrogen to yield a crossliked hydrogel.

### Example 13: Photocuring of acrylate endcapped copolymer of monooleoyl glyceride, succinic anhydride and PEG

0.75 grams of acrylate endcapped polymer of monooleoyl glyceride, succinic anhydride and PEG 400 (25 mol%) synthesized following the procedure of Example 10, and 0.75 grams of PEG diacrylate (Mn = 575) were weighed into a scintillation vial. To this was added 5 milliliter of a solution containing triethanolamine (0.1M), ethyl eosin (0.5 mM) and vinyl pyrrolidone (0.1%). The solution was stirred, poured into an aluminum pan and cured using visible light (514 nm, Xenon light source with fiber optics) to yield a crosslinked hydrogel.

## Claims

1. A composition, comprising: a synthetic, biodegradable, biocompatible polymer comprising the reaction product of a polybasic acid or derivative thereof, a monoglyceride, and a hydrophilic polyol, said polymer having at least one crosslinkable region.

2. The composition of claim 1 wherein said polybasic acid or derivative thereof is succinic acid, succinic anhydride, malic acid, tartaric acid, citric acid, diglycolic acid, diglycolic anhydride, glutaric acid, glutaric anhydride, adipic acid, pimelic acid, suberic acid, sebacic acid, fumaric acid, maleic acid, citraconic acid, itaconic acid, maleic anhydride, a mixed anhydride, an ester, an activated ester or an acid halide.

3. The composition of claim 2 wherein said polybasic acid derivative is succinic anhydride.

4. The composition of claim 2 wherein said polybasic acid is maleic anhydride.

5. The composition of any one of claims 1 to 4 wherein said monoglyceride is monostearoyl glycerol, monopalmitoyl glycerol, monomyrisitoyl glycerol, monocaproyl glycerol, monodecanoyl glycerol, monolauroyl glycerol, monolinoleoyl glycerol or monooleoyl glycerol.

6. The composition of any one of claims 1 to 5 wherein said crosslinkable region comprises an unsaturated polybasic acid.

7. The composition of claim 6 wherein said unsaturated polybasic acid is fumaric acid, maleic acid, citraconic acid or itaconic acid.

8. The composition of any one of claims 1 to 7 wherein said crosslinkable region comprises a multifunctional polybasic acid or hydrophilic polyol.

9. The composition of claim 8 wherein said multifunctional polybasic acid or hydrophilic polyol is malic acid, tartaric acid, citric acid, glycerol, a polygylcerol, a sugar or a sugar alcohol.

10. The composition of any one of claims 1 to 9 wherein said crosslinkable region comprises a crosslinkable end group.

11. The composition of claim 10 wherein said crosslinkable end group is an acrylate, diacrylate, oligoacrylate, methacrylate, dimethacrylate or oligomethoacrylate.

12. The composition of any one of claims 1 to 11 wherein said copolymer comprises the reaction product of said monoglyceride, said hydrophilic polyol, and at least two of said polybasic acids or derivatives thereof.

13. The composition of any one of claims 1 to 11 wherein said copolymer comprises the reaction product of said polybasic acid or derivative thereof, and at least two of said monoglycerides.

14. The composition of any one of claims 1 to 13 further comprising an aliphatic polyester prepared from glycolide, L-lactide, D-lactide, meso-lactide, rac-lactide, ε-caprolactone, trimethylene carbonate, p-dioxanone, 1,4-dioxanone, 1,4-dioxepan-2-one, 1,5-dioxepan-2-one or a substituted derivative thereof.

15. The composition of any one of claims 1 to 14, further comprising an effective amount of a bioactive agent.

16. The composition of claim 15 wherein said bioactive agent is selected from the group consisting of antiinfectives, analgesics, anorexics, antihelmintics, antiarthritics, antiasthmatics, anticonvulsants, antidepressants, antidiuretics, antidiarrheals, antihistamines, antiinflammatory agents, antimigraine preparations, antinauseants, antineoplastics, antiparkinsonism drugs, antipruritics, antipsychotics, antipyretics, antispasmodics, anticholinergics, sympathomimetics, xanthine derivatives, calcium channel blockers, beta-blockers, antiarrhythmics, antihypertensives, diuretics, vasodilators, central nervous system stimulants,decongestants, hormones, steroids, hypnotics, immunosuppressives, muscle relaxants, parasympatholytics, psychostimulants, sedatives, tranquilizers, naturally derived or genetically engineered proteins, polysaccharides, glycoproteins, or lipoproteins, oligonucleotides, antibodies, antigens, cholinergics, chemotherapeutics, hemostatics, clot dissolving agents, radioactive agents and cystostatics.

17. The composition of claim 16 wherein said bioactive agent is risperidone.

18. The composition of claim 16 wherein said bioactive agent is erythropoietin.

19. The composition of claim 16 wherein said bioactive agent is rapamycin.

20. The composition of any one of claims 1 to 19 comprising a hydrogel, said hydrogel comprising said polymer and an amount of water effective to form said hydrogel.

21. The composition any one of claims 1 to 19 comprising a coating, said coating comprising said polymer and a suitable solvent therefore in an amount effective to provide said coating.

22. A synthetic polymer as defined in any one of claims 1 to 19.

23. A medical device comprising a coating, said coating comprising a synthetic polymer as defined in any one of claims 1 to 19.

24. The medical device of claim 23 which is a suture, mesh, tissue engineering scaffold, pin, clip, staple, sheet, foam, anchor, screw, plate, film, suture knot clip, pin, clamp, hook, button, snap, nail, endoscopic instrument, bone substitute, prosthesis, intrauterine device, stent, graft, vertebral disc, extracorporeal tubing for kidney and heart-lung machines or artificial skin.
